(19) Europäisches Patentamt
European Patent Office
Office européen des brevets

(11) EP 3 932 488 B1

(12) EUROPEAN PATENT SPECIFICATION

(45) Date of publication and mention of the grant of the patent:
**11.09.2024 Bulletin 2024/37**

(21) Application number: **20763542.6**

(22) Date of filing: **26.02.2020**

(51) International Patent Classification (IPC):
***A61K 31/7084*** (2006.01) ***A61K 9/08*** (2006.01)
***A61K 47/32*** (2006.01) ***A61K 47/38*** (2006.01)
***A61K 31/7072*** (2006.01) ***A61K 9/00*** (2006.01)
***A61P 27/02*** (2006.01) ***A61P 27/04*** (2006.01)

(52) Cooperative Patent Classification (CPC):
**A61P 27/02; A61K 9/0048; A61K 9/08; A61K 31/6615; A61K 31/7084; A61K 47/32; A61P 27/04**

(86) International application number:
**PCT/JP2020/007638**

(87) International publication number:
**WO 2020/175525 (03.09.2020 Gazette 2020/36)**

(54) **OPHTHALMIC COMPOSITION CONTAINING DIQUAFOSOL OR SALT THEREOF, VINYL-BASED POLYMER AND CELLULOSE-BASED POLYMER**

OPHTHALMISCHE ZUSAMMENSETZUNG MIT DIQUAFOSOL ODER SALZ DAVON, VINYLBASIERTES POLYMER UND CELLULOSEBASIERTES POLYMER

COMPOSITION OPHTALMIQUE CONTENANT DU DIQUAFOSOL OU UN SEL DE CELUI-CI, UN POLYMÈRE À BASE DE VINYLE ET UN POLYMÈRE À BASE DE CELLULOSE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.02.2019 JP 2019033807**

(43) Date of publication of application:
**05.01.2022 Bulletin 2022/01**

(73) Proprietor: **Santen Pharmaceutical Co., Ltd.**
**Kita-ku**
**Osaka-shi**
**Osaka 530-8552 (JP)**

(72) Inventors:
- **MORISHIMA, Kenji**
  **Ikoma-shi, Nara 630-0101 (JP)**
- **ASADA, Hiroyuki**
  **Inukami-gun, Shiga 522-0314 (JP)**
- **MOMOKAWA, Yusuke**
  **Ikoma-shi, Nara 630-0101 (JP)**
- **KAMIMURA, Asuka**
  **Ikoma-shi, Nara 630-0101 (JP)**
- **ENDO, Kenichi**
  **Ikoma-shi, Nara 630-0101 (JP)**

(74) Representative: **Müller-Boré & Partner**
**Patentanwälte PartG mbB**
**Friedenheimer Brücke 21**
**80639 München (DE)**

(56) References cited:
**EP-A1- 1 245 233** **EP-A1- 2 659 894**
**EP-A1- 3 760 207** **WO-A1-2012/090994**
**WO-A1-2018/199180** **WO-A1-2019/168023**
**WO-A1-2019/168023** **WO-A2-01/74330**
**WO-A2-2004/112836** **JP-A- 2003 528 906**
**JP-A- 2018 168 200** **JP-A- 2019 104 727**
**US-A1- 2018 360 746**

- **YAMAGUCHI ET AL: "3% Diquafosol Ophthalmic Solution Promotes Mucin and Tear Fluid", JOURNAL OF THE EYE, MEDICAL AOI PUBLICATION, JP, vol. 32, no. 7, 1 January 2015 (2015-01-01), pages 935 - 942, XP009530097, ISSN: 0910-1810**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- YAMAGUCHI, MASAHIKO ET AL.: "3% Diquafosol Ophthalmic Solution Promotes Mucin and Tear Fluid", JOURNAL OF THE EYE, vol. 32, no. 7, 1 January 2015 (2015-01-01), pages 935 - 942, XP009530097, ISSN: 0910-1810
- GOBBELS, MARTIN ET AL.: "Corneal epithelial permeability of dry eyes before and after treatment with artificial tears.", OPHTHALMOLOGY, vol. 99, no. 6, 1992, pages 873 - 878, XP009091354

**Description**

The present invention relates to an ophthalmic composition comprising diquafosol or a salt thereof, a vinyl-based polymer and a cellulose-based polymer, wherein the vinyl-based polymer comprises polyvinylpyrrolidone and the cellulose-based polymer comprises at least one selected from the group consisting of hydroxyethyl cellulose and methyl cellulse. The present invention further relates to a respective solution-type aqueous eyedrop for use in the treatment of dry eye.

The diquafosol is a purinoceptor agonist called $P^1$, $P^4$-di(uridine-5') tetraphosphate or Up4U, and has a tear secretion promoting action, and the diquafosol sodium, which is a salt thereof, is used for treatment of dry eye as "Diquas (registered trademark) ophthalmic solution 3%" (hereinafter also referred to as "Diquas (registered trademark) ophthalmic solution") (Japanese Patent No. 3652707 (PTL 1), package insert of Diquas (registered trademark) ophthalmic solution 3% (NPL 1)). On the other hand, a dosage of the Diquas (registered trademark) ophthalmic solution is usually a single dose of one drop of the ophthalmic solution 6 times per day (NPL 1). However, in daily life, it is difficult to instill it regularly and frequently, so that there are some patients who do not obtain the expected effects due to poor adherence to instillation.

It has been known that diquafosol or a salt thereof is used in combination with an existing therapeutic agent for dry eye as an attempt to search for a new therapeutic agent for dry eye having a higher tear volume-increasing effect. Japanese Patent Laying-Open No. 2012-077080 (PTL 2) discloses that tear secretion is synergistically promoted by combination use of the diquafosol or a salt thereof with hyaluronic acid, which is a therapeutic agent for dry eye. Japanese Patent Laying-Open No. 2015-160826 (PTL 3) discloses that tear secretion is synergistically promoted by combination use of diquafosol or a salt thereof with rebamipide, which is a therapeutic agent for dry eye. In this context, Yamaguchi *et al.* (Yamaguchi, M. et al.; Journal of the Eye, 32(7); January 1, 2015; pp. 935-942) describes a 3% diquafosol opthalmic solution promoting mucin and tear fluid. Further, EP 2 659 894 A1 describes an ophthalmic solution containing diquafosol, a method for producing the same, and a related method for preventing the formation of insoluble precipitate.

PATENT LITERATURE

PTL 1: Japanese Patent No. 3652707
PTL 2: Japanese Patent Laying-Open No. 2012-077080
PTL 3: Japanese Patent Laying-Open No. 2015-160826

NON PATENT LITERATURE

NPL 1: Package insert of Diquas (registered trademark) ophthalmic solution 3%

An interesting object is to provide a novel ophthalmic composition comprising diquafosol or a salt thereof which has a higher tear volume-increasing effect and enables improvement of adherence to instillation.

The present inventors have found, as a result of diligent experimentation, that an ophthalmic composition comprising diquafosol or a salt thereof, a vinyl-based polymer and a cellulose-based polymer (hereinafter, also referred to as "the present composition") has a high tear volume-increasing effect and the equivalent therapeutic effect even if the instillation frequency of eye drops is reduced as compared with that of the existing Diquas (registered trademark) ophthalmic solution. Furthermore, the present inventors have found that the present composition does not exhibit neurostimulatory and can further improve the feeling of comfort after instillation of ophthalmic solution.

Specifically, the present invention relates to the embodiments characterized in the appended claims.

The references to methods of treatment in this description are to be interpreted as references to the ophthalmic compositions and eye drops of the present invention for use in methods for the prevention or treatment of dry eye.

As is clear from the test results described later, the present composition has a high tear volume-increasing effect. Therefore, the present composition is expected to have a stronger therapeutic effect on dry eye as compared with the case where the existing Diquas (registered trademark) ophthalmic solution is instilled into an eye. Moreover, the existing Diquas (registered trademark) ophthalmic solution is required to be instilled into an eye 6 times a day, and there are some patients who do not obtain the expected effect due to poor adherence to eye drops; however, the present composition is expected to reduce the instillation frequency while having a sufficient therapeutic effect on dry eye and improves adherence to instillation. Furthermore, while the existing Diquas (registered trademark) ophthalmic solution includes diquafosol tetrasodium salt with a concentration of 3% (w/v), the present composition having a lower concentration is expected to demonstrate a similar or greater therapeutic effect on dry eye. Moreover, the present composition does not exhibit neurostimulatory and can improve the feeling of comfort after instillation of ophthalmic solution.

The figures show:

FIG. 1 is a graph illustrating a fluoroscein dyeing score of a cornea after administration of a test drug by eye drop.
FIG. 2 is a diagram illustrating a maximum fluorescence intensity (RFUmax) after addition of diquafosol sodium.

Herein, "(w/v)%" refers to a weight (g) of a component of interest included in 100 mL of the ophthalmic composition of the present invention.
Herein, "PVP" refers to polyvinylpyrrolidone.
Herein, "HEC" refers to hydroxyethyl cellulose.
Herein, "MC" refers to methyl cellulose.
Herein, "CMC-Na" refers to sodium carboxymethyl cellulose.
Herein, "HPMC" refers to hydroxypropyl methylcellulose.
"Diquafosol" is a compound represented by the following chemical structural formula:

[Chemical formula 1]

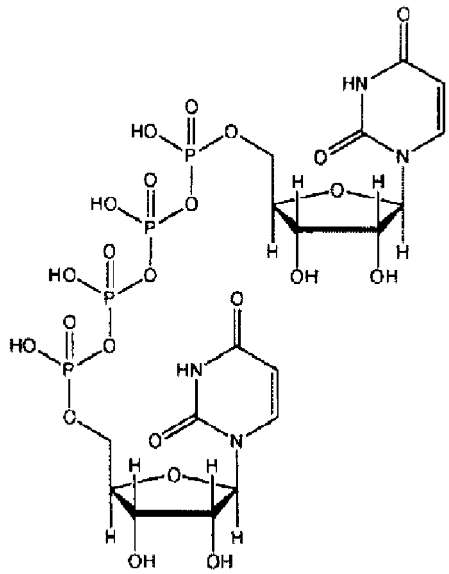

The "salt of diquafosol" is not particularly limited as long as it is a pharmaceutically acceptable salt, and examples thereof includes metal salts with lithium, sodium, potassium, calcium, magnesium, zinc, etc.; salts with inorganic acids, such as hydrochloric acid, hydrobromic acid, hydriodic acid, nitric acid, sulfuric acid, and phosphoric acid; salts with organic acid, such as acetic acid, fumaric acid, maleic acid, succinic acid, citric acid, tartaric acid, adipic acid, gluconic acid, glucoheptonic acid, glucuronic acid, terephthalic acid, methanesulfonic acid, lactic acid, hippuric acid, 1,2-ethanedisulfonic acid, isethionic acid, lactobionic acid, oleic acid, pamoic acid, polygalacturonic acid, stearic acid, tannic acid, trifluoromethanesulfonic acid, benzenesulfonic acid, p-toluenesulfonic acid, lauryl sulfate, methyl sulfate, naphthalene sulfonic acid, and sulfosalicylic acid; quaternary ammonium salts with methyl bromide, and methyl iodide; salts with halogen ions, such as a bromine ion, a chlorine ion, and an iodine ion; salts with ammonia; salts with organic amines, such as triethylenediamine, 2-aminoethanol, 2,2-iminobis(ethanol), 1-deoxy-1-(methylamino)-2-D-sorbitol, 2-amino-2-(hydroxymethyl))-1,3-propanediol, procaine, and N, N-bis(phenylmethyl)-1,2-ethanediamine.
In the present invention, "diquafosol or a salt thereof' also encompasses hydrates and organic solvates of diquafosol (free form) or a salt thereof.
When the "diquafosol or a salt thereof' has crystal polymorphs and crystal polymorph groups (crystal polymorph system), those crystal polymorphs and crystal polymorph groups (crystal polymorph system) are also included within the scope of the present invention. Here, the crystal polymorph group (crystal polymorph system) refers to the whole of the individual crystal forms and the processes thereof at each stage when the crystal shapes change depending on conditions and states in, for example, production, crystallization, and storage of those crystals.
The "diquafosol or a salt thereof" of the present invention is preferably a sodium salt of diquafosol, and the diquafosol tetrasodium salt represented by the following chemical structural formula (also simply referred to as "diquafosol sodium" herein) is particularly preferred.

[Chemical formula 2]

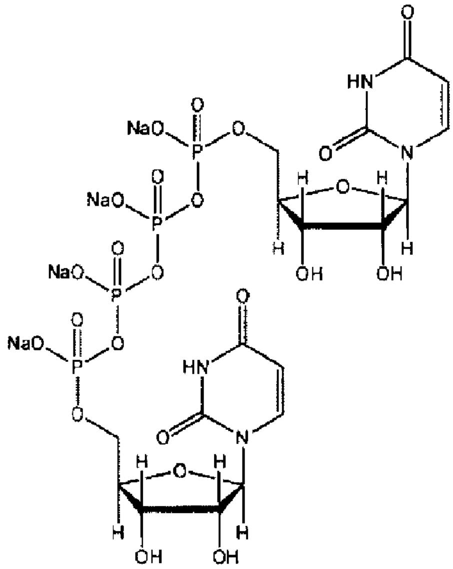

The diquafosol or a salt thereof can be produced by, for example, the method disclosed in Japanese National Patent Publication No. 2001-510484.

The present composition may include another active ingredient in addition to diquafosol or a salt thereof, or may include the diquafosol or a salt thereof as the only active ingredient.

In the present invention, the concentration of diquafosol or a salt thereof is not particularly limited, but for example, it is preferably 0.0001 to 10% (w/v), more preferably 0.001 to 5% (w/v), still more preferably 0.01 to 5% (w/v), even still more preferably 0.1 to 5% (w/v), further even still more preferably 1 to 5% (w/v), and particularly preferably 3% (w/v). More specifically, the concentration is preferably 0.001% (w/v), 0.002% (w/v), 0.003% (w/v), 0.004% (w/v), 0.005%. (w/v), 0.006% (w/v), 0.007% (w/v), 0.008% (w/v), 0.009% (w/v), 0.01% (w/v), 0.02% (w/v), 0.03% (w/v), 0.04% (w/v), 0.05% (w/v), 0.06% (w/v), 0.07% (w/v), 0.08% (w/v), 0.09% (w/v), 0.1% (w/v), 0.2% (w/v), 0.3% (w/v), 0.4% (w/v), 0.5% (w/v), 0.6% (w/v), 0.7% (w/v), 0.8% (w/v), 0.9% (w/v), 1% (w/v), 1.5% (w/v), 2% (w/v), 2.5% (w/v), 3% (w/v), 3.5% (w/v), 4% (w/v), 4.5% (w/v) or 5% (w/v).

In the present invention, the "vinyl-based polymer" comprises polyvinylpyrrolidone.

The molecular weight of the vinyl-based polymer is not particularly limited, but for example, the polymer having a weight-average molecular weight of 2,500 to 3 million, preferably 10,000 to 1.5 million, more preferably 10,000 to 500,000, and still more preferably 10,000 to 400,000, can be used.

The vinyl-based polymer to be used can be a commercially available product, and these compounds can be used singly or in combinations of two or more thereof.

In the present invention, the concentration of the vinyl-based polymer is not particularly limited, and may be, for example, 0.001% (w/v) or more, preferably 0.001 to 10% (w/v), more preferably 0.01 to 10% (w/v), still more preferably 0.05 to 10% (w/v), even still more preferably 0.1 to 10% (w/v), and further even still more preferably 0.1 to 5% (w/v), and particularly preferably 1 to 5% (w/v).

In the present invention, polyvinylpyrrolidone is a polymer compound obtained by polymerizing N-vinyl-2-pyrrolidone. The K value of polyvinylpyrrolidone used in the present invention is preferably 17 or more, more preferably 17 to 90, still more preferably 25 to 90, even still more preferably 30 to 90, and particularly preferably 30. Examples include polyvinylpyrrolidone K17, polyvinylpyrrolidone K25, polyvinylpyrrolidone K30, polyvinylpyrrolidone K40, polyvinylpyrrolidone K50, polyvinylpyrrolidone K60, polyvinylpyrrolidone K70, polyvinylpyrrolidone K80, polyvinylpyrrolidone K85, polyvinylpyrrolidone K90, and polyvinylpyrrolidone K120. The K value of polyvinylpyrrolidone is a value characteristic of the viscosity that correlates with the molecular weight, and is a numerical value calculated by substituting the relative viscosity value (25°C) measured by a capillary viscometer to the following Fikentscher equation (1):
[Expression 1]

$$K=\frac{1.5\log\eta_{rel}-1}{0.15+0.003c}+\frac{[300c\log\eta_{rel}+2(c+1.5\log\eta_{rel})]^{1/2}}{0.15c+0.003c^{2}} \qquad (1)$$

In equation (1), $\eta_{rel}$ is a relative viscosity of the polyvinylpyrrolidone aqueous solution to water, and c is a concentration (%) of polyvinylpyrrolidone in the polyvinylpyrrolidone aqueous solution.

In the present invention, a polyvinylpyrrolidone may be used alone, or two or more polyvinylpyrrolidones having different K values may be arbitrarily combined and used.

In the present invention, the concentration of polyvinylpyrrolidone is not particularly limited, and may be, for example, 0.001% (w/v) or more, preferably 0.001 to 10% (w/v), and more preferably 0.01 to 10% (w/v), still more preferably 0.05 to 10% (w/v), even still more preferably 0.1 to 10% (w/v), and further even still more preferably 0.1 to 5 % (w/v), and particularly preferably 1 to 5% (w/v).

The present composition further comprises a cellulose-based polymer. Cellulose is a fibrous polymer in which D-glucopyranoses are linked via a $\beta 1 \rightarrow 4$ glucoside bond. In the present invention, the "cellulose-based polymer" comprises at least one selected from the group consisting of methyl cellulose and hydroxyethyl cellulose. Hydroxyethyl cellulose is preferred.

In the present invention, a cellulose-based polymer may be used alone, or two or more cellulose-based polymers may be arbitrarily combined and used.

The concentration of the cellulose-based polymer in the present composition is not particularly limited, and for example, it is preferably 0.0001 to 5% (w/v), more preferably 0.001 to 3% (w/v), still more preferably 0.01 to 2% (w/v), and even still more preferably 0.1 to 1% (w/v).

The combination of the vinyl-based polymer and the cellulose-based polymer that are used in the present composition is preferably a combination of polyvinylpyrrolidone and hydroxyethyl cellulose, and more preferably a combination of polyvinylpyrrolidone having a K value of 30 (polyvinylpyrrolidone K30) and hydroxyethyl cellulose. When the polyvinylpyrrolidone having a K value of 30 and the hydroxyethyl cellulose are combined for use, the concentration of the polyvinylpyrrolidone having a K value of 30 is preferably 0.1 to 5% (w/v) and the concentration of the hydroxyethyl cellulose is preferably 0.01 to 2% (w/v), for example.

The amount of polyvinylpyrrolidone and hydroxyethyl cellulose incorporated in the present composition can also be such that the viscosity of the present composition falls within a preferred range described later.

The present composition may include the polyvinylpyrrolidone, preferably polyvinylpyrrolidone having a K value of 30 as only vinyl-based polymer, and hydroxyethyl cellulose as only cellulose-based polymer.

Using a generally employed technique, pharmaceutically acceptable additives can be further incorporated in the present composition as needed. For example, buffer agents, such as sodium phosphate, sodium hydrogen phosphate, sodium hydrogen phosphate hydrate, sodium dihydrogen phosphate, sodium acetate, epsilon-aminocaproic acid; isotonic agents, such as calcium chloride, sodium chloride, potassium chloride, concentrated glycerin; stabilizers, such as sodium edetate, sodium edetate hydrate; surfactants, such as polysorbate; antioxidants, such as ascorbic acid; preservatives, such as benzalkonium chloride and chlorhexidine gluconate; pH adjustors, such as hydrochloric acid and sodium hydroxide, can be selected and added as needed. These additives may be used singly or in any combination of two or more thereof.

The present composition may include preservatives, such as benzalkonium chloride and chlorhexidine gluconate, as described above, but may include no preservatives or substantially no preservatives.

The pH of the present composition is not limited to a specific value as long as it is within a range acceptable for a drug. However, the pH of the present composition is preferably 8 or less, more preferably in the range of 4 to 8, still more preferably in the range of 5 to 8, even still more preferably in the range of 6 to 8, and particularly preferably in the vicinity of 7.

In the present invention, the "ophthalmic composition" refers to a composition for use in prevention and/or treatment of eye diseases, etc. Examples of the dosage form thereof includes eye drops, eye ointments, injections, and ointments (for example, which can be administered to a skin of eyelids), and eye drops are preferred. Here, the eye drop is synonymous with an ophthalmic solution or an ophthalmic drug, and eye drops for contact lenses is also encompassed by the definition of eye drops.

The present composition is preferably an aqueous ophthalmic composition including water as a solvent (a substrate), and more preferably an aqueous eye drop.

The present composition may be a solution-type eye drop or may be a suspension-type eye drop, depending on the active ingredients and the properties and contents of the additives.

The viscosity of the present composition is not particularly limited as long as it is within a range acceptable for a drug, and the viscosity is adjusted so as to be preferably in the range of 1 to 500 mPa·s, more preferably in the range of 1 to 100 mPa·s, still more preferably 1 to 50 mPa·s, and even still more preferably in the range of 1 to 40 mPa·s, and is measured with a rotational viscometer (25°C; shear rate of $50s^{-1}$).

The osmotic pressure of the present composition is not limited to a specific value as long as it is within the range acceptable for a drug. However, the osmotic pressure of the present composition is preferably 2 or less, more preferably 0.5 to 2, still more preferably 0.7 to 1.6, even still more preferably 0.8 to 1.4, and particularly preferably 0.9 to 1.2.

The present composition can be contained and stored in a container made of various materials. For example,

containers made of polyethylene, polypropylene, etc., can be used. When the present composition is an eye drop, it is contained in an eyedrop container, more specifically, in a "multi-dose type eyedrop container" or a "unit-dose type eyedrop container".

In the present invention, the "multi-dose type eyedrop container" is an eyedrop container provided with a container body and a cap that can be attached to the container body, and the cap can be freely opened and resealed. The multi-dose type eyedrop container usually contains multiple doses of ophthalmic solution for use for a certain period of time.

On the other hand, the "unit-dose type eyedrop container" is an eyedrop container having a cap fused and sealed at the bottle mouth, and is intended to be used by breaking and opening the fused portion between the cap and the bottle-shaped body when used. The unit-dose type eyedrop container contains ophthalmic solution for one or several uses. In general, the ophthalmic solution contained in the unit-dose type eye drops container include no or substantially no preservatives, such as benzalkonium chloride.

The dose regimen of the present composition can be appropriately changed according to the dosage form, the severity of a symptom of a patient to be dosed, the age and the body weight of the patient, the doctor's judgment, and the like. For example, when an eye drop is selected as the dosage form, the present composition can be instilled into an eye 1 to 6 times a day, preferably 1 to 4 times a day, more preferably 1 to 2 times a day, at intervals of one day to one week, in a dose of 1 to 5 drops, preferably 1 to 3 drops, more preferably 1 to 2 drops, especially preferably 1 drop each time. Here, more specifically, the instillation frequency is, for example, preferably 6 times a day, 5 times a day, 4 times a day, 3 times a day, 2 times a day or 1 time a day, more preferably 6 times a day, 4 times a day, 3 times a day or 2 times a day, still more preferably 4 times a day or 3 times a day, especially preferably 3 times a day.

When the concentration of diquafosol or a salt thereof in the present composition is 3% (w/v), the present composition can be instilled into an eye 6 times a day, 5 times a day, 4 times a day, 3 times a day, 2 times a day or 1 time a day, preferably 6 times a day, 4 times a day, 3 times a day or 2 times a day, more preferably 4 times a day or 3 times a day, especially preferably 3 times a day, in a dose of 1 to 5 drops, preferably 1 to 3 drops, more preferably 1 to 2 drops, especially preferably 1 drop each time.

One drop is preferably about 0.1 to 30 μL, more preferably about 0.5 to 20 μL, and still more preferably about 1 to 15 μL.

The present composition is effective as a preventive or therapeutic agent for dry eye. The dry eye is defined as "a chronic disease of tear fluid and keratoconjunctival epithelium caused by various factors and accompanied by eye discomfort and visual abnormalities", and keratoconjunctivitis sicca (KCS) is encompassed by dry eye. In the present invention, the occurrence of dry eye symptoms caused by wearing soft contact lenses is also encompassed by the dry eye.

The dry eye symptoms include subjective symptoms, such as dry eye, eye discomfort, eye fatigue, eye dullness, photophobia, eye pain, and blurred vision, as well as objective symptoms, such as congestion and keratoconjunctival epithelial disorder.

There are many unclear points regarding the etiology of dry eye, but it has been reported that the disease is caused by Sjogren's syndrome; congenital alacrima; sarcoidosis; Graft Versus Host Disease (GVHD) by bone marrow transplantation; ocular pemphigoid; Stevens. Johnson syndrome; Lacrimal obstruction caused by tracoma, etc.; diabetes; reduced reflex secretion caused by keratorefractive surgery (LASIK: Laser (-assisted) in situ Keratomilisis), etc.; meibomian gland dysfunction; decrease in an oil layer caused by blepharitis, etc.; poor blinking or abnormalities of eyelid closure caused by eyeball protrusion, rabbit eye, etc.; decrease in mutin secretion from embryonic cells; VDT (Visual Display Therminals) work, etc.

Moreover, the present composition can be instilled in the eyes of dry eye patients wearing soft contact lenses. Here, installing eye drops into the eyes of dry eye patients wearing soft contact lenses refer to installing the eye drops while the soft contact lenses are worn on the corneas of the dry eye patient.

## Examples

The results of pharmacological tests and formulation examples are shown below, but these are for a better understanding of the present invention and do not limit the scope of the present invention.

### [Test 1]

Using normal male white rabbits, the time-course of tear volume after instillation of the present composition was evaluated.

(Drug preparation method)

Ophthalmic solution 1:

An ophthalmic solution 1 was prepared according to the formulation table shown in Table 1 (the unit is g/100 mL in Table 1). Specifically, diquafosol sodium (9 g), sodium hydrogen phosphate hydrate (0.6 g), sodium edetate hydrate (0.03 g) and sodium chloride (1.35 g) were dissolved in sterilized purified water to make 50 mL of a 6-fold concentrated solution. Moreover, after mixing 10 mL of the 6-fold concentrated solution and 5 mL of sterilized purified water and then dissolving PVP K30 (1.2 g), the pH was adjusted to 7 by appropriately adding a pH adjustor, and sterilized purified water was added to obtain 20 mL of a 3-fold concentrated solution. Hydroxyethyl cellulose (15 g) was dissolved in sterilized purified water to make the total amount of 1500 g, and then high-pressure steam sterilization (121°C, 20 minutes) was carried out to obtain a 1.00% (w/w) hydroxyethyl cellulose solution. Ophthalmic solution 1 was prepared by adding 4 mL of the 3-fold concentrated solution to 3.6 g of the 1.00% (w/w) hydroxyethyl cellulose solution, adding sterile purified water to adjust the total volume to 12 mL, and then adding a pH adjustor as appropriate to adjust a pH to 7.

Ophthalmic solution 2 (Reference solution):

An ophthalmic solution 2 was prepared according to the formulation table shown in Table 1. Specifically, diquafosol sodium (9 g), sodium hydrogen phosphate hydrate (0.6 g), sodium edetate hydrate (0.03 g) and sodium chloride (1.35 g) were dissolved in sterilized purified water to make 50 mL of a 6-fold concentrated solution. Moreover, after mixing 10 mL of the 6-fold concentrated solution and 5 mL of sterilized purified water, the pH was adjusted to 7 by appropriately adding a pH adjustor, and sterilized purified water was added to obtain 20 mL of a 3-fold concentrated solution. PVP K90 (4 g) was dissolved in sterilized purified water to make the total amount of 100 g, and then high-pressure steam sterilization (121°C, 20 minutes) was carried out to obtain a 4.00% (w/w) PVP K90 solution. Ophthalmic solution 2 was prepared by adding 4 mL of the 3-fold concentrated solution to 6.0 g of the 4.00% (w/w) PVP K90 solution, adding sterile purified water to adjust the total volume to 12 mL, and then adding a pH adjustor as appropriate to adjust a pH to 7.

Ophthalmic solution 3 (Reference solution):

An ophthalmic solution 3 was prepared according to the formulation table shown in Table 1. Specifically, diquafosol sodium (9 g), sodium hydrogen phosphate hydrate (0.6 g), sodium edetate hydrate (0.03 g) and sodium chloride (1.35 g) were dissolved in sterilized purified water to make 50 mL of a 6-fold concentrated solution. Moreover, after mixing 10 mL of the 6-fold concentrated solution and 5 mL of sterilized purified water and then dissolving PVP K30 (1.2 g), the pH was adjusted to 7 by appropriately adding a pH adjustor, and sterilized purified water was added to obtain 20 mL of a 3-fold concentrated solution. Ophthalmic solution 3 was prepared by adding sterile purified water to 4 mL of the 3-fold concentrated solution to make the total amount of 12 mL and then adding a pH adjustor as appropriate to adjust a pH to 7.

Ophthalmic solution 4 (Reference solution):

An ophthalmic solution 4 was prepared according to the formulation table shown in Table 1. Specifically, diquafosol sodium (9 g), sodium hydrogen phosphate hydrate (0.6 g), sodium edetate hydrate (0.03 g) and sodium chloride (1.35 g) were dissolved in sterilized purified water to make 50 mL of a 6-fold concentrated solution. Moreover, after mixing 10 mL of the 6-fold concentrated solution and 5 mL of sterilized purified water, the pH was adjusted to 7 by appropriately adding a pH adjustor, and sterilized purified water was added to obtain 20 mL of a 3-fold concentrated solution. Hydroxyethyl cellulose (15 g) was dissolved in 1500 mL of sterilized purified water, and then high-pressure steam sterilization (121°C, 20 minutes) was carried out to obtain a 1.00% (w/w) hydroxyethyl cellulose solution. Ophthalmic solution 4 was prepared by adding 4 mL of the 3-fold concentrated solution to 3.6 g of the 1.00% (w/w) hydroxyethyl cellulose solution, adding sterile purified water to adjust the total volume to 12 mL, and then adding a pH adjustor as appropriate to adjust a pH to 7.

Ophthalmic solution 5 (Reference solution):

An ophthalmic solution 5 was prepared according to the formulation table shown in Table 1. Specifically, diquafosol sodium (9 g), sodium hydrogen phosphate hydrate (0.6 g), sodium edetate hydrate (0.03 g) and sodium chloride (1.35 g) were dissolved in sterilized purified water to make 50 mL of a 6-fold concentrated solution. Moreover, after mixing 10 mL of the 6-fold concentrated solution and 5 mL of sterilized purified water, the pH was adjusted to 7 by

appropriately adding a pH adjustor, and sterilized purified water was added to obtain 20 mL of a 3-fold concentrated solution. Ophthalmic solution 5 was prepared by adding sterile purified water to 4 mL of the 3-fold concentrated solution to adjust the total amount to 12 mL and then adding a pH adjustor as appropriate to adjust a pH to 7.

Ophthalmic solution 6:

An ophthalmic solution 6 was prepared according to the formulation table shown in Table 2 (the unit is g/100 mL in Table 2). Specifically, diquafosol sodium (18 g), sodium hydrogen phosphate hydrate (1.2 g), and sodium edetate hydrate (0.06 g) were dissolved in sterilized purified water to make 100 mL of a 6-fold concentrated solution. Moreover, after mixing 5 mL of the 6-fold concentrated solution and 5 mL of sterilized purified water and then dissolving PVP K25 (0.9 g) and sodium chloride (0.135 g), the pH was adjusted to 7 by appropriately adding a pH adjustor, and sterilized purified water was added to obtain 15 mL of a 2-fold concentrated solution. Hydroxyethyl cellulose (15 g) was dissolved in sterilized purified water to make the total amount of 1500 g, and then high-pressure steam sterilization (121°C, 20 minutes) was carried out to obtain a 1.00% (w/w) hydroxyethyl cellulose solution. Ophthalmic solution 6 was prepared by adding 7.5 mL of the 2-fold concentrated solution to 3.75 g of the 1.00% (w/w) hydroxyethyl cellulose solution, adding sterile purified water to adjust the total volume to 15 mL, and then adding a pH adjustor as appropriate to adjust a pH to 7.

Ophthalmic solution 7:

An ophthalmic solution 7 was prepared according to the formulation table shown in Table 2. Specifically, diquafosol sodium (18 g), sodium hydrogen phosphate hydrate (1.2 g), and sodium edetate hydrate (0.06 g) were dissolved in sterilized purified water to make 100 mL of a 6-fold concentrated solution. Moreover, after mixing 20 mL of the 6-fold concentrated solution and 20 mL of sterilized purified water and then dissolving PVP K30 (2.4 g) and sodium chloride (0.54 g), the pH was adjusted to 7 by appropriately adding a pH adjustor, and sterilized purified water was added to obtain 60 mL of a 2-fold concentrated solution. Hydroxyethyl cellulose (15 g) was dissolved in sterilized purified water to make the total amount of 1500 g, and then high-pressure steam sterilization (121°C, 20 minutes) was carried out to obtain a 1.00% (w/w) hydroxyethyl cellulose solution. Ophthalmic solution 7 was prepared by adding 50 mL of the 2-fold concentrated solution to 25 g of the 1.00% (w/w) hydroxyethyl cellulose solution, adding sterile purified water to adjust the total volume to 100 mL, and then adding a pH adjustor as appropriate to adjust a pH to 7.

Ophthalmic solution 8 (Reference solution):

An ophthalmic solution 8 was prepared according to the formulation table shown in Table 2. Specifically, diquafosol sodium (18 g), sodium hydrogen phosphate hydrate (1.2 g), and sodium edetate hydrate (0.06 g) were dissolved in sterilized purified water to make 100 mL of a 6-fold concentrated solution. Moreover, ophthalmic solution 8 was prepared by mixing 2.5 mL of the 6-fold concentrated solution and 5 mL of sterilized purified water, then dissolving a PVP K60 45% aqueous solution (0.67 g) and sodium chloride (0.068 g), adjusting a pH to 7 by appropriately adding a pH adjustor, and adding sterilized purified water to make a volume of 15 mL.

Ophthalmic solutions 9 to 11:

An ophthalmic solution 9 was prepared according to the formulation table shown in Table 3 (the unit is g/100 mL in Table 3). Specifically, diquafosol sodium (9 g), sodium hydrogen phosphate hydrate (0.6 g), sodium edetate hydrate (0.03 g) and sodium chloride (1.35 g) were dissolved in sterilized purified water to make 50 mL of a 6-fold concentrated solution. Moreover, after mixing 10 mL of the 6-fold concentrated solution and 5 mL of sterilized purified water and then dissolving PVP K30 (1.2 g), the pH was adjusted to 7 by appropriately adding a pH adjustor, and sterilized purified water was added to obtain 20 mL of a 3-fold concentrated solution. Methyl cellulose (2 g) was dissolved in sterilized purified water to make the total amount of 100 g, and then high-pressure steam sterilization (121°C, 20 minutes) was carried out to obtain a 2.00% (w/w) methyl cellulose solution. Ophthalmic solution 9 was prepared by adding 4 mL of the 3-fold concentrated solution to 3.0 g of the 2.00% (w/w) methyl cellulose solution, adding sterile purified water to adjust the total volume to 12 mL, and then adding a pH adjustor as appropriate to adjust a pH to 7.

Ophthalmic solutions 10 and 11 (Reference solutions) were prepared by the same method as for ophthalmic solution 9.

The viscosity of the prepared ophthalmic solutions 1 to 11 was measured by using a rotational viscometer Kinexus pro+ at a temperature of 25°C and a shear rate of $50s^{-1}$.

(Test method and drug administration method)

Normal male white rabbits (23 rabbits, 46 eyes in total) were instilled with Benokiseal (registered trademark) Ophthalmic Solution 0.4% (manufactured by Santen Pharmaceutical Co., Ltd.) to be subjected to local anesthesia. Three minutes later, a Schirmer test strip (manufactured by AYUMI Pharmaceutical Corporation) was inserted into the lower eyelid, and one minute after the insertion, the strip was removed and the length of the wet portion (tear volume) was read, which was designated as the previous value. Next, each of ophthalmic solutions 1 to 11 was instilled once (4 rabbits per group, 8 eyes, and only ophthalmic solution 5 was instilled to 16 rabbits, 32 eyes). Three minutes before inserting a Schirmer test strip (manufactured by AYUMI Pharmaceutical Corporation) into the lower eyelid, the rabbits were instilled with Benokiseal (registered trademark) Ophthalmic Solution 0.4% (manufactured by Santen Pharmaceutical Co., Ltd.) and subjected to local anesthesia. After 60 minutes from instillation of each ophthalmic solution, a Schirmer test strip (manufactured by AYUMI Pharmaceutical Corporation) was inserted into the lower eyelid, and taken out 1 minute after the insertion, and the length of the wet portion (tear volume) was read.

(Evaluation method)

The change in tear volume before and after instillation of the ophthalmic solution, which was defined as Δ tear volume (mm/min), was calculated.

(Test results)

Tables 1 to 3 show the amount of Δ tear volume (mm/min) after 60 minutes from instillation (each value is the average value of 8 eyes, and, for only ophthalmic solution 5, is the average value of 32 eyes.). Moreover, the tear volume-increasing effect of the present composition was evaluated and ranked according to the following criteria.

+++: Δ tear volume (mm/min) after 60 minutes from instillation is 4 mm/min or more

++: Δ tear volume (mm/min) after 60 minutes from instillation is 1 mm/min or more and less than 4 mm/min

+: Δ tear volume (mm/min) after 60 minutes from instillation is more than 0 mm/min to less than 1 mm/min

-: Δ tear volume (mm/min) after 60 minutes from instillation is 0 mm/min or less.

[Table 1]

| Ophthalmic solution | 1 | 2 | 3 | 4 | 5 |
|---|---|---|---|---|---|
| Diquafosol sodium | 3 | 3 | 3 | 3 | 3 |
| PVP K30 | 2 | - | 2 | - | - |
| PVP K90 | - | 2 | - | - | - |
| HEC | 0.3 | - | - | 0.3 | - |
| Sodium hydrogen phosphate hydrate | 0.2 | 0.2 | 0.2 | 0.2 | 0.2 |
| Sodium edetate hydrate | 0.01 | 0.01 | 0.01 | 0.01 | 0.01 |
| Sodium chloride | 0.45 | 0.45 | 0.45 | 0.45 | 0.45 |
| pH adjustor | q. s. | q. s. | q. s. | q. s. | q.s. |
| pH | 7.0 | 7.0 | 7.0 | 7.0 | 7.0 |
| Viscosity (mPa·s) | 37.8 | 7.4 | 1.4 | 26.9 | 1.0 |
| Δ tear volume (mm/min) after 60 minutes from instillation | 4.9 | 4.4 | 0.4 | -0.6 | 0.3 |
| Evaluation | +++ | +++ | + | - | + |

As shown in the results of Table 1 above, ophthalmic solution including PVP K30 (ophthalmic solution 3) did not exhibit the tear volume-increasing effect after 60 minutes from instillation similarly to ophthalmic solution not including

it (ophthalmic solution 5). HEC is generally used as a thickener as well, and ophthalmic solution including HEC (ophthalmic solution 4) had a relatively high viscosity though but did not exhibit the tear volume-increasing effect after 60 minutes from instillation similarly to ophthalmic solution 5. On the other hand, ophthalmic solution including PVP K30 and HEC (ophthalmic solution 1) surprisingly exhibited the extremely higher tear volume-increasing effect than ophthalmic solutions 3 to 5.

[Table 2]

| Ophthalmic solution | 6 | 7 | 8 |
|---|---|---|---|
| Diquafosol sodium | 3 | 3 | 3 |
| PVP K25 | 3 | - | - |
| PVP K30 | - | 2 | - |
| PVP K60 | - | - | 2 |
| HEC | 0.25 | 0.25 | - |
| Sodium hydrogen phosphate hydrate | 0.2 | 0.2 | 0.2 |
| Sodium edetate hydrate | 0.01 | 0.01 | 0.01 |
| Sodium chloride | 0.45 | 0.45 | 0.45 |
| pH adjustor | q. s. | q. s. | q.s. |
| pH | 7.0 | 7.0 | 7.0 |
| Viscosity (mPa·s) | 23.4 | 22.4 | 3.0 |
| Δ tear volume (mm/min) after 60 minutes from instillation | 2.8 | 4.3 | 4.4 |
| Evaluation | ++ | +++ | +++ |

As shown in the results of Table 2 above, ophthalmic solution including PVP and HEC (ophthalmic solutions 6 and 7) had the high tear volume-increasing effect even when the K value of PVP was 25 in addition to the case where it was 30.

[Table 3]

| Ophthalmic solution | 9 | 10 | 11 |
|---|---|---|---|
| Diquafosol sodium | 3 | 3 | 3 |
| PVP K30 | 2 | 2 | 2 |
| HEC | | | |
| MC | 0.5 | - | - |
| HPMC | - | 0.5 | - |
| CMC-Na | - | - | 0.5 |
| Sodium hydrogen phosphate hydrate | 0.2 | 0.2 | 0.2 |
| Sodium edetate hydrate | 0.01 | 0.01 | 0.01 |
| Sodium chloride | 0.45 | 0.45 | 0.45 |
| pH adjustor | q. s. | q. s. | q.s. |
| pH | 7.0 | 7.0 | 7.0 |
| Viscosity (mPa·s) | 10.2 | 10.5 | 6.4 |
| Δ tear volume (mm/min) after 60 minutes from instillation | 1.8 | 0.3 | -0.1 |
| Evaluation | ++ | + | - |

As shown in the results of Table 3 above, ophthalmic solution including PVP K30 and MC (ophthalmic solution

9) had the high tear volume-increasing effect. On the other hand, ophthalmic solution including PVP K30 and HPMC (ophthalmic solution 10) or ophthalmic solution including PVP K30 and CMC-Na (ophthalmic solution 11) did not exhibit the tear volume-increasing effect.

[Test 2]

The rat extraorbital lacrimal gland excision model is widely used as a model for evaluating the therapeutic effect of corneal epithelial disorder caused by dry eye, and is also used as a model for evaluating the therapeutic effect of $P2Y_2$ receptor agonists (Invest. Ophthalmol. Vis. Sci., 42 (1), 96-100 (2001)). Using the dry eye model, it was examined whether or not an improvement effect on corneal epithelial disorder was obtained by administration by eye drop of the present composition.

(How to fabricate dry eye model)

Using male SD rats, the rat extraorbital lacrimal gland excision model was prepared according to the method of Fujihara et al. (Invest. Ophthalmol. Vis. Sci., 42 (1), 96-100 (2001)). Specifically, the rats were each administered with somnopentyl to be subjected to general anesthesia, and then the extraorbital lacrimal gland was removed to induce corneal epithelial disorder.

(Sample preparation method)

Ophthalmic solution A:

An ophthalmic solution A was prepared according to the formulation table shown in Table 4 (the unit is g/100 mL in Table 4). Specifically, sodium hydrogen phosphate hydrate (4 g) and sodium chloride (9 g) were dissolved in sterilized purified water to obtain 200 mL of a 10-fold buffer solution. Moreover, diquafosol sodium (15 g) was dissolved in sterilized purified water to obtain a total amount of 50 g of a 30% diquafosol sodium aqueous solution. Hydroxyethyl cellulose (2 g) was dissolved in sterilized purified water to make a total amount of 200 g, and then high-pressure steam sterilization (121°C, 40 minutes) was carried out to obtain a 1.00% (w/w) hydroxyethyl cellulose solution. 50 mL of sterilized purified water, 20 mL of the 10-fold buffer solution, 20 mL of the 30% diquafosol sodium aqueous solution, and PVP K30 (4 g) were mixed and dissolved, the pH was adjusted to 7 by using a pH adjustor to obtain a total volume of 100 mL of a 2-fold concentrated solution. Ophthalmic solution A was prepared by adding 50 mL of the 2-fold concentrated solution to 25 g of the 1.00% (w/w) hydroxyethyl cellulose solution, adding sterile purified water to adjust the total volume to 100 mL, and adding a pH adjustor as appropriate to adjust the pH to 7.

Ophthalmic solution B (Reference solution):

An ophthalmic solution B was prepared according to the formulation table shown in Table 4 (the unit is g/100 mL in Table 4). Specifically, sodium hydrogen phosphate hydrate (4 g) and sodium chloride (9 g) were dissolved in sterilized purified water to obtain a total volume of 200 mL of a 10-fold buffer solution. Hydroxyethyl cellulose (2 g) was dissolved in sterilized purified water to make a total amount of 200 g, and then high-pressure steam sterilization (121°C, 40 minutes) was carried out to obtain a 1.00% (w/w) hydroxyethyl cellulose solution. 50 mL of sterilized purified water, 20 mL of the 10-fold buffer solution, sodium chloride (0.76 g) and PVP K30 (4 g) were mixed and dissolved, the pH was adjusted to 7 by using a pH adjustor, and a total volume of 100 mL of a 2-fold concentrated solution was obtained. Ophthalmic solution B was prepared by adding 50 mL of the 2-fold concentrated solution to 25 g of the 1.00% (w/w) hydroxyethyl cellulose solution, adding sterile purified water to adjust the total volume to 100 mL, and adding a pH adjustor as appropriate to adjust the pH to 7. Ophthalmic solution B are substrates of ophthalmic solution A.

Ophthalmic solution X (Reference solution):

An ophthalmic solution X, "Diquas (registered trademark) ophthalmic solution 3%" (manufactured by Santen Pharmaceutical Co., Ltd.), which had been used as a therapeutic drug for dry eye, was used. Ophthalmic solution X include 30 mg of diquafosol sodium as an active ingredient per 1 mL of water, and also include potassium chloride, sodium chloride, chlorhexidine gluconate liquid, sodium hydrogen phosphate hydrate, sodium edetate hydrate, and a pH adjustor as additives.

[Table 4]

| Ophthalmic solution | A | B |
|---|---|---|
| Diquafosol sodium | 3 | - |
| PVP K30 | 2 | 2 |
| HEC | 0.25 | 0.25 |
| Sodium hydrogen phosphate hydrate | 0.2 | 0.2 |
| Sodium chloride | 0.45 | 0.45 |
| pH adjustor | q. s. | q. s. |
| pH | 7 | 7 |

(Test method and drug administration method)

Ophthalmic solution A, ophthalmic solution B, and ophthalmic solution X were administered as follows to the rats induced by the corneal epithelial disorder.

- Ophthalmic solution A, twice a day administration group: Ophthalmic solution A were instilled in both eyes twice a day for 4 weeks (6 rats per group, 12 eyes).
- Ophthalmic solution A, 3 times per day administration group: Ophthalmic solution A were instilled in both eyes 3 times per day for 4 weeks (6 rats per group, 12 eyes).
- Ophthalmic solution A, 4 times per day administration group: Ophthalmic solution A were instilled in both eyes 4 times per day for 4 weeks (6 rats per group, 12 eyes).
- Ophthalmic solution X, 6 times per day administration group: Ophthalmic solution X were instilled in both eyes 6 times per day for 4 weeks (6 rats per group, 12 eyes).
- Ophthalmic solution B, 4 times per day administration group (substrate administration group): Ophthalmic solution B were instilled in both eyes 4 times per day for 4 weeks (6 rats per group, 12 eyes).

Among the rats induced by corneal epithelial disorder, those that had not been instilled for 4 weeks were included in non-instillation group (4 rats per group, 8 eyes).

Four weeks after the start of instillation, the damaged portion of the cornea was dyed with fluorescein, and corneal epithelial disorder was determined according to the method of Murakami et al. (New Ophthalmology, 21 (1), 87-90 (2004)). Namely, for each of the upper portion, middle portion and lower portion of the cornea, the degree of dyeing with fluorescein was scored according to the following criteria, and the average value of the total of those scores was calculated. An intermediate score with an increment value of 0.5 was set between the scores of 0, 1, 2, and 3.

(Evaluation criteria)

0: Undyed
1: Sparse dyeing and separation of each dotted dyed portion
2: Medium dyeing and adjacency of each fractional part of dotted dyed portion
3: Dense dyeing and adjacency of each dotted dyed portion.

(Result)

FIG. 1 shows a graph of the calculated scores of fluorescein dyeing degrees for each group. The score is the average value + standard error of 8 or 12 cases.

As is clear from FIG. 1, the improvement on the score of fluorescein dyeing degree was observed in the group administered with ophthalmic solution A 3 times per day and 4 times per day as compared with the group administered with the substrate drops (ophthalmic solution B administered 4 times per day), which was equivalent to the improvement on the score of fluorescein dyeing degree in the group administered with ophthalmic solution X 6 times per day. Ophthalmic solution X is used for treatment of dry eye as the Diquas (registered trademark) ophthalmic solution 3%, and the instillation

frequency is 6 times per day. Therefore, there are some patients who do not obtain the expected effect due to poor adherence to instillation. In contrast, the present composition reduces the instillation frequency to 3 times per day or 4 times per day while having a sufficient therapeutic effect on dry eye, and is expected to improve adherence to instillation.

[Test 3]

The stimulativeness of diquafosol sodium to peripheral nerves in the coexistence of PVP was examined.

(Sample preparation method)

Formulation solution 1 (Reference solution):

A formulation solution 1 was prepared according to the formulation table shown in Table 5 (the unit is g/100 mL in Table 5). Specifically, sodium chloride (8.5 g) and sodium hydrogen phosphate hydrate (2 g) were dissolved in sterile purified water, a pH adjustor was added to adjust the pH to 7.5, and the total volume was adjusted to 100 mL to obtain a 10-fold buffer solution. PVP K30 (16 g) was dissolved in sterilized purified water to adjust the total volume to 200 mL, and an 8% PVP K30 aqueous solution was obtained. 2 mL of the 10-fold buffer solution and 5 mL of the 8% PVP K30 aqueous solution were weighed, the total volume was adjusted to 20 mL with sterile purified water, and the pH was adjusted to 7.5 by using a pH adjustor to obtain formulation solution 1.

Formulation solution 2 (Reference solution):

A formulation solution 2 was prepared according to the formulation table shown in Table 5. Specifically, sodium chloride (8.5 g) and sodium hydrogen phosphate hydrate (2 g) were dissolved in sterile purified water, a pH adjustor was added to adjust the pH to 7.5, and the total volume was adjusted to 100 mL to obtain a 10-fold buffer solution. 2 mL of the 10-fold buffer solution and PVP K90 (0.4 g) were dissolved in sterile purified water, the pH was adjusted to 7.5 by using a pH adjustor, and the total volume was adjusted to 20 mL to obtain formulation solution 2.

Formulation solution 3:

A formulation solution 3 was prepared according to the formulation table shown in Table 5. Specifically, sodium chloride (8.5 g) and sodium hydrogen phosphate hydrate (2 g) were dissolved in sterile purified water, a pH adjustor was added to adjust the pH to 7.5, and the total volume was adjusted to 100 mL to obtain a 10-fold buffer solution was obtained. PVP K30 (16 g) was dissolved in sterilized purified water to adjust the total volume to 200 mL, to obtain an 8% PVP K30 aqueous solution. 4 mL of the 10-fold buffer solution and 10 mL of the 8% PVP K30 aqueous solution were weighed, the total volume was adjusted to 20 mL with sterile purified water, and the pH was adjusted to 7.5 by using a pH adjustor to obtain a 2-fold concentrated solution of formulation solution 3. Hydroxyethyl cellulose (1 g) was dissolved in sterilized purified water to make the total amount of 100 g, and then high-pressure steam sterilization (121°C, 25 minutes) was carried out to obtain a 1.00% (w/w) hydroxyethyl cellulose solution. Sterile purified water was added to 6 g of the 1.00% (w/w) hydroxyethyl cellulose solution and 10 mL of the 2-fold concentrated solution of formulation 3 to adjust the total volume to 20 mL

Formulation solution 4 (Reference solution):

A formulation solution 4 was prepared according to the formulation table shown in Table 5. Specifically, sodium chloride (8.5 g) and sodium hydrogen phosphate hydrate (2 g) were dissolved in sterile purified water, a pH adjustor was added to adjust the pH to 7.5, and the total volume was adjusted to 100 mL to obtain a 10-fold buffer solution. 2 mL of the 10-fold buffer and sodium chondroitin sulfate (0.06 g) were added to sterile purified water, the pH was adjusted to 7.5 by using a pH adjustor, and after confirming complete dissolution, the total volume was adjusted to 20 mL to obtain formulation solution 4.

Formulation solutions 5 to 7 (Reference solutions):

According to the formulation table shown in Table 5, formulation solutions 5 to 7 were prepared in the same manner as for formulation solution 4.

[Table 5]

| | 1 | 2 | 3 | 4 | 5 | 6 | 7 |
|---|---|---|---|---|---|---|---|
| Sodium chloride | 0.85 | 0.85 | 0.85 | 0.85 | 0.85 | 0.80 | 0.85 |
| Sodium hydrogen phosphate hydrate | 0.20 | 0.20 | 0.20 | 0.20 | 0.20 | 0.20 | 0.20 |
| PVP K30 | 2.00 | - | 2.00 | - | - | - | - |
| PVP K90 | - | 2.00 | - | - | - | - | - |
| HEC | - | - | 0.30 | - | - | - | - |
| Sodium chondroitin sulfate | - | - | - | 0.30 | - | - | - |
| HPMC | - | - | - | - | 0.30 | - | - |
| CVP | - | - | - | - | - | 0.30 | - |
| CMC-Na | - | - | - | - | - | - | 0.30 |
| pH adjustor | q.s. | q.s. | q.s. | q.s. | q.s. | q.s. | q.s. |
| pH | 7.5 | 7.5 | 7.5 | 7.5 | 7.5 | 7.5 | 7.5 |

(Test method)

Cultured peripheral nerve cells (rat dorsal root ganglion neurons, purchased from Lonza Japan Ltd.) were incubated in a buffer solution (FLIPR Calcium 6 Assay Kit, Molecular Devices, LLC.) including intracellular fluorescent calcium indicator dyes. 40% of the total buffer solution was replaced with each of the aforementioned formulation solutions. The non-stimulation group and the stimulated control group were treated with the buffer solutions instead of the formulation solutions in the same manner. After allowing to stand at room temperature, the fluorescence measurement of the calcium indicator dyes with an elapse of time was started by using a fluorescent plate reader. Diquafosol sodium (final concentration: 0.3%) was added after 60 seconds from the start, and the measurement of fluorescence intensity was continued.

(Evaluation method)

The maximum fluorescence intensity (RFUmax) after addition of diquafosol sodium was calculated relative to the fluorescence intensity (RFU) immediately before the addition as 100%.

(Test results)

The results are shown in FIG. 2. In the stimulated control group and the formulation solutions 4 to 7, RFU was increased after the addition of diquafosol sodium, and RFUmax of 103.5% or more was recorded. On the other hand, all of RFUmax was less than 101% in each group of the formulation solutions 1 to 3 including PVPs.

(Discussion)

Peripheral nerve cells that received any stimulus generate an action potential and become excited, and the signal of stimulus converted to the action potential is then transmitted to the central nervous system. The action potential is a change in cell membrane potential caused by the intracellular influx of cations including calcium ions. Therefore, an increase in intracellular calcium ion concentration has been widely used experimentally as an index indicating the excitatory state of nerve cells. When peripheral nerve cells were exposed to diquafosol sodium, the fluorescence intensity of intracellular calcium ions was rapidly increased, indicating that the nerve cells received diquafosol sodium as a stimulus and became excited. Similar stimulus responses were observed in each group of PVP-free polymer formulation solutions 4 to 7 as Comparative Examples, and any polymer of chondroitin sodium sulfate, HPMC, CVP or CMC-Na exhibited no effect on neurostimulatory with diquafosol sodium. On the other hand, in the case of formulation solutions 1 to 3 including PVPs, no increase in signal of intracellular calcium ions after the addition of diquafosol sodium was exhibited. Namely, it was indicated that the diquafosol sodium coexisting with PVP did not exhibit neurostimulatory, and that the addition of PVP and the addition of PVP as well as HEC improved the feeling of comfort after instillation of the eye drop.

[Preparation Example]

The drugs of the present invention will be described in more detail by way of formulation examples, but the present invention is not limited to these formulation examples.

(Formulation example 1: eye drop (3% (w/v))

| | |
|---|---|
| In 100 mL | |
| Diquafosol sodium | 3 g |
| Sodium hydrogen phosphate hydrate | 0.01 to 0.5 g |
| Sodium chloride | 0.01 to 1 g |
| Sodium edetate hydrate | 0.0001 to 0.1 g |
| Polyvinylpyrrolidone | 0.0001 to 10 g |
| Hydroxyethyl cellulose | 0.0001 to 5 g |
| pH adjustor | q.s. |

The aforementioned eye drop can be prepared by adding diquafosol sodium and other above components to sterilized purified water and mixing them sufficiently.

The present composition has a high tear volume-increasing effect. Therefore, the present composition is expected to have a stronger therapeutic effect on dry eye as compared with the case where the existing Diquas (registered trademark) ophthalmic solution is administered by eye drop. Moreover, the existing Diquas (registered trademark) ophthalmic solution needs to be instilled 6 times per day, and there are some patients who do not obtain the expected effect due to poor adherence to instillation; however, the present composition reduces the instillation frequency while having a sufficient therapeutic effect on dry eye, and is expected to exhibit improvement of adherence to instillation. Furthermore, while the existing Diquas (registered trademark) ophthalmic solution includes diquafosol tetrasodium salt with a concentration of 3%(w/v) , the present composition having a lower concentration is expected to demonstrate a similar or greater therapeutic effect on dry eye. Moreover, the present composition does not exhibit neurostimulatory and can improve feeling of comfort after instillation of eye drops.

## Claims

1. An ophthalmic composition comprising diquafosol or a salt thereof, a vinyl-based polymer and a cellulose-based polymer, wherein the vinyl-based polymer comprises polyvinylpyrrolidone and the cellulose-based polymer comprises at least one selected from the group consisting of hydroxyethyl cellulose and methyl cellulose.

2. The ophthalmic composition according to claim 1, comprising polyvinylpyrrolidone having a K value of 17 or more.

3. The ophthalmic composition according to claim 1 or 2, comprising polyvinylpyrrolidone having a K value of 17 to 90.

4. The ophthalmic composition according to any one of claims 1 to 3, comprising polyvinylpyrrolidone having a K value of 30.

5. The ophthalmic composition according to any one of claims 1 to 4, wherein a concentration of the vinyl-based polymer is 0.001 % (w/v) or more.

6. The ophthalmic composition according to any one of claims 1 to 5, wherein a concentration of the cellulose-based polymer is 0.0001 to 5% (w/v).

7. The ophthalmic composition according to any one of claims 1 to 6, wherein a concentration of the diquafosol or a salt thereof is 0.0001 to 10% (w/v).

8. The ophthalmic composition according to any one of claims 1 to 7, wherein a concentration of the diquafosol or a

salt thereof is 0.01 to 5% (w/v).

**9.** The ophthalmic composition according to any one of claims 1 to 8, wherein a concentration of the diquafosol or a salt thereof is 1 to 5% (w/v).

**10.** The ophthalmic composition according to any one of claims 1 to 9, wherein a concentration of the diquafosol or a salt thereof is 3% (w/v).

**11.** The ophthalmic composition according to any one of claims 1 to 10, wherein the ophthalmic composition is an eye drop.

**12.** The ophthalmic composition according to any one of claims 1 to 11, wherein the ophthalmic composition is aqueous.

**13.** The ophthalmic composition according to any one of claims 1 to 12, wherein the ophthalmic composition is a solution-type composition.

**14.** The ophthalmic composition according to any one of claims 1 to 13, wherein the viscosity is 1 to 500 mPa·s at 25°C.

**15.** The ophthalmic composition according to any one of claims 1 to 14, wherein the salt of diquafosol is diquafosol sodium.

**16.** The ophthalmic composition according to any one of claims 1 to 15 for use in the prevention or treatment of dry eye.

**17.** The ophthalmic composition for use according to claim 16, wherein the ophthalmic composition is instilled into an eye 1 to 6 times a day in a dose of 1 to 5 drops each time.

**18.** The ophthalmic composition for use according to claim 16 or 17, wherein the ophthalmic composition is instilled into an eye 2 to 4 times a day in a dose of 1 or 2 drops each time.

**19.** The ophthalmic composition for use according to any one of claims 16 to 18, wherein the ophthalmic composition is instilled into an eye 3 or 4 times a day in a dose of 1 or 2 drops each time.

**20.** A solution-type aqueous eye drop for use in the treatment of dry eye comprising diquafosol sodium with a concentration of 3%(w/v), hydroxyethyl cellulose and polyvinylpyrrolidone having a K value of 30, wherein the eye drop is instilled into an eye 3 times a day in a dose of 1 or 2 drops each time.

## Patentansprüche

**1.** Ophthalmische Zusammensetzung, umfassend Diquafosol oder ein Salz davon, ein Polymer auf Vinylbasis und ein Polymer auf Cellulosebasis, wobei das Polymer auf Vinylbasis Polyvinylpyrrolidon umfasst und das Polymer auf Cellulosebasis mindestens eines aus der Gruppe, bestehend aus Hydroxyethylcellulose und Methylcellulose, umfasst.

**2.** Ophthalmische Zusammensetzung nach Anspruch 1, umfassend Polyvinylpyrrolidon mit einem K-Wert von 17 oder mehr.

**3.** Ophthalmische Zusammensetzung nach Anspruch 1 oder 2, umfassend Polyvinylpyrrolidon mit einem K-Wert von 17 bis 90.

**4.** Ophthalmische Zusammensetzung nach einem der Ansprüche 1 bis 3, umfassend Polyvinylpyrrolidon mit einem K-Wert von 30.

**5.** Ophthalmische Zusammensetzung nach einem der Ansprüche 1 bis 4, wobei eine Konzentration des Polymers auf Vinylbasis 0,001 % (w/v) oder mehr beträgt.

**6.** Ophthalmische Zusammensetzung nach einem der Ansprüche 1 bis 5, wobei eine Konzentration des Polymers auf Cellulosebasis 0,0001 bis 5 % (w/v) beträgt.

7. Ophthalmische Zusammensetzung nach einem der Ansprüche 1 bis 6, wobei eine Konzentration des Diquafosols oder eines Salzes davon 0,0001 bis 10 % (w/v) beträgt.

8. Ophthalmische Zusammensetzung nach einem der Ansprüche 1 bis 7, wobei eine Konzentration des Diquafosols oder eines Salzes davon 0,01 bis 5% (w/v) beträgt.

9. Ophthalmische Zusammensetzung nach einem der Ansprüche 1 bis 8, wobei eine Konzentration des Diquafosols oder eines Salzes davon 1 bis 5 % (w/v) beträgt.

10. Ophthalmische Zusammensetzung nach einem der Ansprüche 1 bis 9, wobei eine Konzentration des Diquafosols oder eines Salzes davon 3% (w/v) beträgt.

11. Ophthalmische Zusammensetzung nach einem der Ansprüche 1 bis 10, wobei die ophthalmische Zusammensetzung ein Augentropfen ist.

12. Ophthalmische Zusammensetzung nach einem der Ansprüche 1 bis 11, wobei die ophthalmische Zusammensetzung wässrig ist.

13. Ophthalmische Zusammensetzung nach einem der Ansprüche 1 bis 12, wobei die ophthalmische Zusammensetzung eine Zusammensetzung vom Lösungstyp ist.

14. Ophthalmische Zusammensetzung nach einem der Ansprüche 1 bis 13, wobei die Viskosität 1 bis 500 mPa·s bei 25°C beträgt.

15. Ophthalmische Zusammensetzung nach einem der Ansprüche 1 bis 14, wobei das Salz von Diquafosol Diquafosol-Natrium ist.

16. Ophthalmische Zusammensetzung nach einem der Ansprüche 1 bis 15 zur Verwendung bei der Vorbeugung oder Behandlung von trockenen Augen.

17. Ophthalmische Zusammensetzung zur Verwendung nach Anspruch 16, wobei die ophthalmische Zusammensetzung 1- bis 6-mal pro Tag in einer Dosis von jeweils 1 bis 5 Tropfen in ein Auge eingeträufelt wird.

18. Ophthalmische Zusammensetzung zur Verwendung nach Anspruch 16 oder 17, wobei die ophthalmische Zusammensetzung 2- bis 4-mal pro Tag in einer Dosis von jeweils 1 oder 2 Tropfen in ein Auge eingeträufelt wird.

19. Ophthalmische Zusammensetzung zur Verwendung nach einem der Ansprüche 16 bis 18, wobei die ophthalmische Zusammensetzung 3- oder 4-mal pro Tag in einer Dosis von jeweils 1 oder 2 Tropfen in ein Auge eingeträufelt wird.

20. Wässrige Augentropfen vom Lösungstyp zur Verwendung bei der Behandlung von trockenen Augen, umfassend Diquafosol-Natrium mit einer Konzentration von 3% (w/v), Hydroxyethylcellulose und Polyvinylpyrrolidon mit einem K-Wert von 30, wobei die Augentropfen dreimal pro Tag in einer Dosis von jeweils 1 oder 2 Tropfen in ein Auge eingeträufelt werden.

## Revendications

1. Composition ophtalmique comprenant du diquafosol ou un sel de celui-ci, un polymère à base de vinyle et un polymère à base de cellulose, dans laquelle le polymère à base de vinyle comprend de la polyvinylpyrrolidone et le polymère à base de cellulose comprend au moins un composé sélectionné parmi le groupe constitué de l'hydroxyéthylcellulose et la méthylcellulose.

2. Composition ophtalmique selon la revendication 1, comprenant de la polyvinylpyrrolidone ayant une valeur de K de 17 ou plus.

3. Composition ophtalmique selon la revendication 1 ou 2, comprenant de la polyvinylpyrrolidone ayant une valeur de K de 17 à 90.

4. Composition ophtalmique selon l'une quelconque des revendications 1 à 3, comprenant de la polyvinylpyrrolidone ayant une valeur K de 30.

5. Composition ophtalmique selon l'une quelconque des revendications 1 à 4, dans laquelle une concentration en le polymère à base de vinyle est de 0,001 % (p/v) ou plus.

6. Composition ophtalmique selon l'une quelconque des revendications 1 à 5, dans laquelle une concentration en le polymère à base de cellulose est de 0,0001 à 5 % (p/v).

7. Composition ophtalmique selon l'une quelconque des revendications 1 à 6, dans laquelle une concentration en le diquafosol ou un sel de celui-ci est de 0,0001 à 10 % (p/v).

8. Composition ophtalmique selon l'une quelconque des revendications 1 à 7, dans laquelle une concentration en le diquafosol ou un sel de celui-ci est de 0,01 à 5 % (p/v).

9. Composition ophtalmique selon l'une quelconque des revendications 1 à 8, dans laquelle une concentration en le diquafosol ou un sel de celui-ci est de 1 à 5 % (p/v).

10. Composition ophtalmique selon l'une quelconque des revendications 1 à 9, dans laquelle une concentration en le diquafosol ou un sel de celui-ci est de 3 % (p/v).

11. Composition ophtalmique selon l'une quelconque des revendications 1 à 10, dans laquelle la composition ophtalmique est une goutte oculaire.

12. Composition ophtalmique selon l'une quelconque des revendications 1 à 11, dans laquelle la composition ophtalmique est aqueuse.

13. Composition ophtalmique selon l'une quelconque des revendications 1 à 12, dans laquelle la composition ophtalmique est une composition de type solution.

14. Composition ophtalmique selon l'une quelconque des revendications 1 à 13, dans laquelle la viscosité est de 1 à 500 mPa·s à 25 °C.

15. Composition ophtalmique selon l'une quelconque des revendications 1 à 14, dans laquelle le sel de diquafosol est du diquafosol sodique.

16. Composition ophtalmique selon l'une quelconque des revendications 1 à 15 destinée à être utilisée dans la prévention ou le traitement de la sécheresse oculaire.

17. Composition ophtalmique destinée à être utilisée selon la revendication 16, dans laquelle la composition ophtalmique est instillée dans un oeil 1 à 6 fois par jour à une dose de 1 à 5 gouttes à chaque fois.

18. Composition ophtalmique destinée à être utilisée selon la revendication 16 ou 17, dans laquelle la composition ophtalmique est instillée dans un oeil 2 à 4 fois par jour à une dose de 1 ou 2 gouttes à chaque fois.

19. Composition ophtalmique destinée à être utilisée selon l'une quelconque des revendications 16 à 18, dans laquelle la composition ophtalmique est instillée dans un oeil 3 ou 4 fois par jour à une dose de 1 ou 2 gouttes à chaque fois.

20. Goutte oculaire aqueuse de type solution destinée à être utilisée dans le traitement de la sécheresse oculaire comprenant du diquafosol sodique avec une concentration de 3 % (p/v), de l'hydroxyéthylcellulose et de la polyvinylpyrrolidone ayant une valeur de K de 30, dans laquelle la goutte oculaire est instillée dans un oeil 3 fois par jour à une dose de 1 ou 2 gouttes à chaque fois.

FIG.1

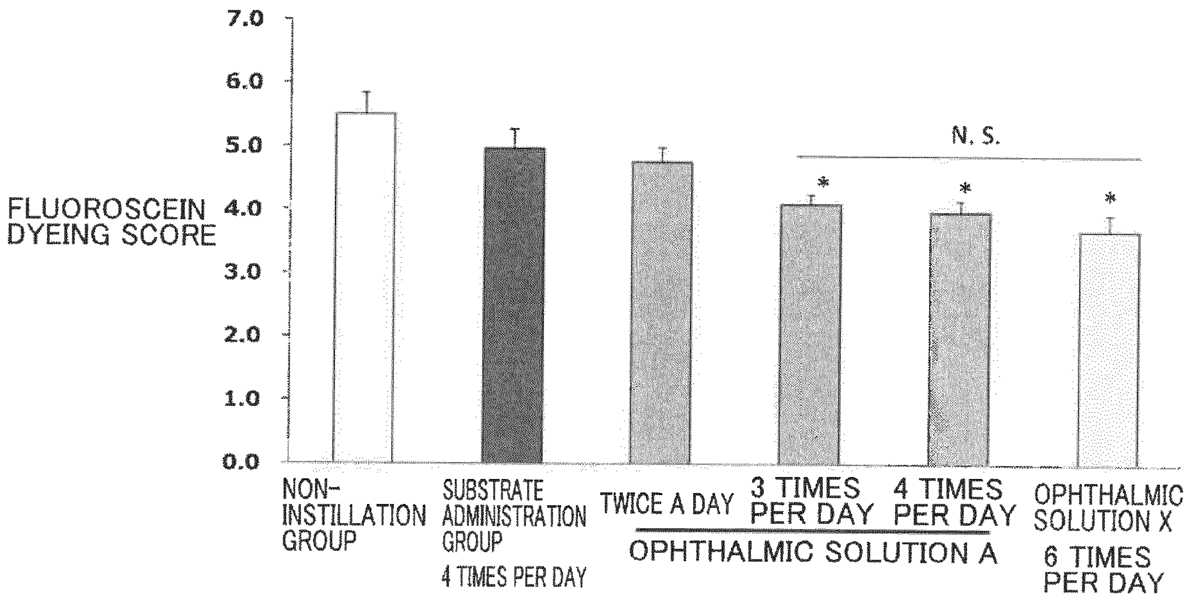


MEAN+SE (N=8 or 12)

*:P<0.05 VS SUBSTRATE ADMINISTRATION GROUP (STUDENT T-TEST)

N. S.: NO SIGNIFICANT DIFFERENCE (TUKEY MULTIPLE COMPARISON TEST)

FIG.2

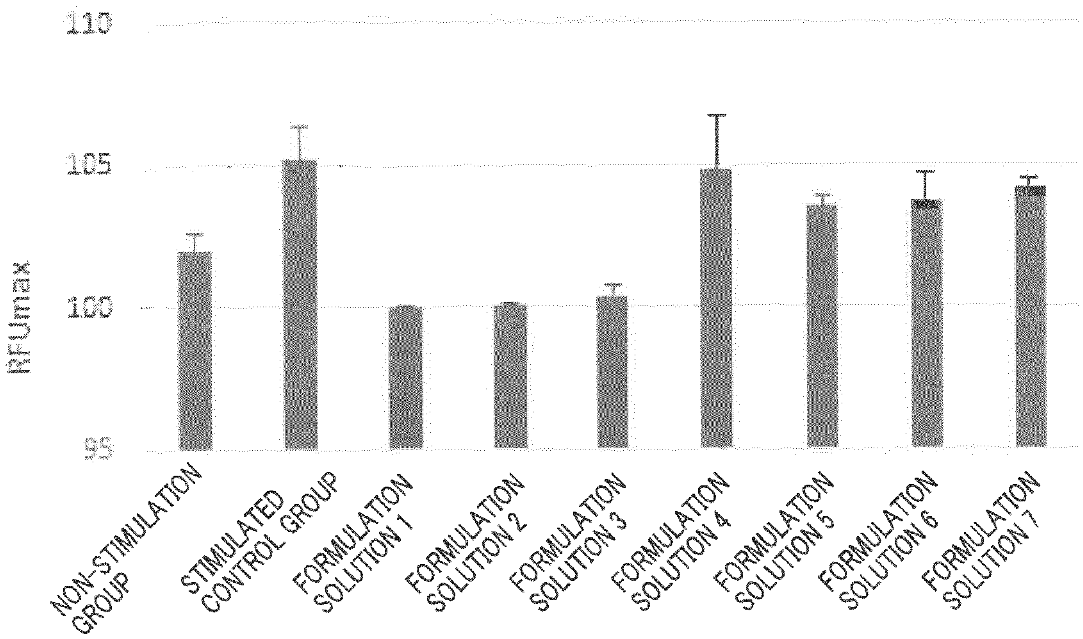


EACH VALUE INDICATES AVERAGE VALUE + STANDARD ERROR (N= 3 TO 5)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 3652707 B
- JP 2012077080 A
- JP 2015160826 A
- EP 2659894 A1
- JP 2001510484 A


### Non-patent literature cited in the description


- **YAMAGUCHI, M. et al.** *Journal of the Eye,* 01 January 2015, vol. 32 (7), 935-942
- *Invest. Ophthalmol. Vis. Sci.,* 2001, vol. 42 (1), 96-100
- **FUJIHARA et al.** *Invest. Ophthalmol. Vis. Sci.,* 2001, vol. 42 (1), 96-100
- **MURAKAMI et al.** *New Ophthalmology,* 2004, vol. 21 (1), 87-90